Europäisches Patentamt

European Patent Office

Office européen des brevets

(19)

(11) Veröffentlichungsnummer: **0 155 669 B1**

(12) **EUROPÄISCHE PATENTSCHRIFT**

(45) Veröffentlichungstag der Patentschrift: 29.05.91

(51) Int. Cl.⁵: **C12N 11/14**, C02F 3/10

(21) Anmeldenummer: **85103163.3**

(22) Anmeldetag: **19.03.85**

(54) Mit Mikroorganismen bewachsene poröse anorganische Träger, Verfahren zur Immobilisierung von Mikroorganismen und dafür geeignete Trägerkörper.

(30) Priorität: **23.03.84 DE 3410650**

(43) Veröffentlichungstag der Anmeldung:
**25.09.85 Patentblatt 85/39**

(45) Bekanntmachung des Hinweises auf die Patenterteilung:
**29.05.91 Patentblatt 91/22**

(84) Benannte Vertragsstaaten:
**AT BE CH DE FR GB IT LI LU NL SE**

(56) Entgegenhaltungen:
EP-A- 0 054 987
EP-A- 0 183 207
FR-A- 2 243 011
GB-A- 2 004 300

CHEMICAL ABSTRACTS, Band 101, Nr. 4, 23. Juli 1984, Seite 256, Zusammenfassung Nr. 27758u, Columbus Ohio, US; A. AIVASIDIS et al.: "A 'glass sponge' as a storage material for bacteria-wastewater treatment without oxygen"

(73) Patentinhaber: **Schott Glaswerke**
Hattenbergstrasse 10
W-6500 Mainz(DE)BE CH DE FR IT LI LU NL

SE AT

Patentinhaber: **Forschungszentrum Jülich GmbH**
Postfach 1913
W-5170 Jülich(DE)BE CH DE FR IT LI LU NL
SE AT

Patentinhaber: **CARL-ZEISS-STIFTUNG**
Schott Glaswerke Hattenbergstrasse 10
W-6500 Mainz 1(DE)GB

(72) Erfinder: **Aivasidis, Alexander, Dr.**
Kopernikusstrasse 33
W-5170 Jülich(DE)
Erfinder: **Wandrey, Christian, Prof.**
Wolfshovener Strasse 139
W-5170 Jülich-Stetternich(DE)
Erfinder: **Kiefer, Werner, Dr.**
Jupiterweg 19
W-6500 Mainz-Finthen(DE)

(74) Vertreter: **Dr. Fuchs, Dr. Luderschmidt Dipl.-Phys. Seids, Dr. Mehler Patentanwälte**
Abraham-Lincoln-Strasse 7
W-6200 Wiesbaden(DE)

**Beschreibung**

Die Erfindung bezieht sich auf ein Verfahren zur Immobilisierung von Mikroorganismen und tierischen Zellen, insbesondere für anaerobe Prozesse, an porösen anorganischen Trägerkörpern und sie umfaßt die danach erhältlichen mit Mikroorganismen bewachsenen Trägerkörper sowie die zur Immobilisierung geeigneten Trägerkörper.

Die Immobilisierung von Mikroorganismen und Zellmaterialien an festen Trägern ist ein Mittel, um solche Materialien am gewünschten Ort räumlich anzureichen. Dies ist insbesondere bei biotechnologischen Prozessen von Bedeutung.

Sowohl aerobe als auch anaerobe Prozesse der Biotechnologie sollen mit möglichst hohen Raumzeitausbeuten (umgesetztes Substrat pro Volumen- und Zeiteinheit) ablaufen. Diese Forderung ist um so einfacher zu erfüllen, je höher die Konzentration der aktiven Zellen ist, denen gleichzeitig Produkt- und Katalysatorrolle zukommt.

Hohe Zellkonzentrationen werden bei aeroben Systemen, deren Zellwachstum praktisch ungehemmt erfolgt, ohne weiteres erreicht. Bei anaeroben Systemen unterliegt dagegen das Zellwachstum von vorn herein einer Limitierung, so daß nur relativ geringe Biomassekonzentrationen erzielt werden. Insbesondere in jüngerer Zeit wird jedoch gerade den anaeroben Systemen wegen der günstigen Energiebilanz (Biogasbildung einerseits und Wegfall von Energie für den bei aeroben Systemen notwendigen Sauerstoffeintrag) besondere Aufmerksamkeit geschenkt. Man hat erkannt, daß sich mit solchen Systemen ohne großen Energieaufwand aus billigen Substraten vielfach wertvolle Disproportionierungsprodukte herstellen lassen. Ein besonderes Beispiel dafür ist die anaerobe Aufbereitung hochkonzentrierter Abwässer, bei der bis zu 95 % der organischen Schmutzfracht in Biogas umgewandelt werden, wobei nur 3-4 % in Biomasse überführt wird.

Das geringe Mikroorganismenwachstum bei anaeroben Systemen macht insbesondere hier eine Biomasserückhaltung und Aufkonzentrierung erforderlich, die aber auch bei aeroben Systemen etwa zur Lösung von Trennproblemen interessant sein kann.

Die Immobilisierung von Mikroorganismen an festen Trägern ist daher bereits seit langem geübt und untersucht worden. Dabei wurden insbesondere aus der Umwelt bekannte oder billig verfügbare Trägermaterialien wie Sand, Lavagestein, Keramik, Aktivkohle, Anthrazit, Glas usw. untersucht, mit denen eine mehr oder minder gute Immobilisierung der Mikroorganismen erreicht wird.

In jüngerer Zeit sind insbesondere mehr organische Trägermaterialien in das Blickfeld gerückt: So wurde von I. Karube et al. (Biotechnol. Bioeng. Bd. 22 (1980) Seiten 847-857) die Immobilisierung von methanogehen Bakterien an Polyacrylamidgel, Agargel und Collagenmembranen untersucht, von denen nur Agargel als geeignet gefunden wurde, wobei allerdings auf das geringe Diffusionsvermögen der Nährstoffe und von Methan durch den Agargel hingewiesen wird.

P. Scherer et al. (Biotechnol. Bioeng. Bd. 23 (1981) Seiten 1057-1067) berichten über die Immobilisierung von Methanosarcina barkeri an $Ca^{2+}$-vernetztem Alginatnetzwerk, das in Form von Pellets mit unterschiedlichem Durchmesser von 1,2 bis 3,7 mm untersucht wurde. Dabei wurde entgegen den Angaben von P.S.J. Cheetham et al. (Biotechnol. Bioeng. Bd. 21 (1979) 2155ff.), nach dem ein verzögerter Substrattransport in die Alginatpellets hinein erfolgen soll, kein Unterschied der Aktivität der Mikroorganismen abhängig vom Pelletdurchmesser gefunden.

Von B. Kressdorf et al. wurde auf dem 5. Symp. Techn. Mikrobiol. Sept. 82, Berlin, über die Immobilisierung von Hefen und Bakterien durch Ca-Alginatgel berichtet. Dabei wurden vergleichende Untersuchungen mit unterschiedlichen Trägertypen angestellt; als besonders brauchbar werden mit Biomasse beladene vernetzte Alginatkugeln von hoher Festigkeit mit einem Durchmesser unter 1 mm angegeben.

Vergleichende Untersuchungen wurden schließlich von P. Huysman et al. (Biotechn. Letters, Vol.5 Nr.9 (1983) Seiten 643-648) durchgeführt, und zwar wurden als Trägermaterialien zum einen als "nicht poröse Materialien" bezeichnete Teilchen von etwa 5 mm Größe aus Sepiolith, Zeolith, Argex (feuer-expandierter Ton mit Oberflächenporen von 0,1 - 7,5 $\mu$m) und Glasperlen untersucht und zum anderen als "poröse Materialien" Naturschwamm mit einer Porosität von etwa 50 % und Porengrößen von $\mu$m bis cm sowie unvernetzter Polyurethanschaumstoff mit einer Porosität von etwa 30 % und Poren von $\mu$m bis mm sowie schließlich unterschiedliche Sorten von vernetztem Polyurethanschaumstoff mit einer Porosität von 97 % und einheitlichen Porendurchmessern von (a) 2,21 mm; (b) 430 $\mu$m und (c) 270 $\mu$m. Schließlich wurde in die Untersuchungen auch mit Bentonit beschichteter Polyurethanschaum mit einheitlicher Porengröße von 430 $\mu$m einbezogen.

Dabei wurde festgestellt, daß von den "nicht-porösen Materialien" lediglich Sepiolith zu einer brauchbaren Koloniebildung Anlaß gibt, der bei kristallographischer Untersuchung eine Zusammensetzung aus feinen Nadelbündeln mit einer Länge von 2 $\mu$m zeigt. Diese Nadelbündel zeigten eine Vielzahl von Spalten in der Größe der Bakterien.

Als besondere geeignet erwiesen sich jedoch vornehmlich die porösen Materialien, wobei als vorherschender Faktor die große Porosität und Porengröße erkannt wurden. Dabei ergab insbesondere das Material mit 430 $\mu$m Poren und einer Porosität von 97 % mit und ohne Bentonit-Beschichtung günstige Ergebnisse, und zwar wurden mit diesem vernetzten Polyurethanschaumstoff in einer Periode von 2 Wochen ca. 25 Liter Biogas (65 % Methan) pro Liter Reaktor und Tag erzeugt.

In der DE-OS 28 39 580 werden schließlich eine Reihe von porösen Trägermaterialien, insbesondere Glasfritten für die Immobilisierung von Mikroorganismen angegeben, deren Poren zu $\geq$ 70 % wenigstens so groß wie die kleinste Hauptabmessung der Mikroorganismen aber kleiner als das 4- bzw. 5-fache der größten Abmessung (bei Hefezellen bzw. Bakterien) sein sollen. Dabei wurde sowohl unporöses Borosilicatglas als auch Glasfritten mit Poren über 20 $\mu$m als deutlich schlechter befunden als Material mit Poren unter 20 $\mu$m.

Trotz der zahlreichen unterschiedlichen Untersuchungen über Trägermaterialien und Entwicklung zum Teil recht brauchbarer Trägerkörper ist jedoch das Problem der Immobilisierung von Mikroorganismen noch nicht allseits befriedigend gelöst, da jeweils unterschiedliche Aspekte wie Dichte, Abriebfestigkeit, Stabilität, Langzeitverhalten, Benetzbarkeit und desgleichen problematisch sind und das generelle Ziel einer besonders hohen Effektivität der immobilisierten Biomasse noch nicht erreicht ist.

Aufgabe der Erfindung ist es daher, einen Weg für die Immobilisierung von Mikroorganismen und Zellmaterialien zu finden, so daß eine hohe Aufkonzentierung der Biomasse bei gleichzeitiger hoher Bioaktivität erreicht werden kann.

Aufgabe der Erfindung ist es außerdem, einen geeigneten Trägerkörper zu finden, der mit Mikroorganismen bewachsen sein kann und einen freien Flüssigkeits- und Gasaustausch vom Trägerinneren zur Umgebung hin zuläßt.

Diese Aufgaben werden dadurch gelöst,daß man einen Immmobilisierungsverfahren der eingangs genannten Art als Trägerkörper poröse Sinterkörper mit einer Porendoppelstruktur verwendet mit porositätsbestimmenden durchgehenden Makroporen, die einen freien Flüssigkeits- und Gasaustausch vom Trägerinneren zur Umgebung hin zulassen und mit die Makroporenwände durchsetzenden offenen Mikroporen, deren Größe im Bereich der Mikroorganismen- bzw. Zellgröße liegt.

Es hat sich überraschenderweise gezeigt, daß die Verwendung von porösen Sinterkörpern der vorstehend genannten Art zu einer erheblichen Steigerung der Effektivität des Bioprozesses führt. Maßgebend dafür ist die spezielle Struktur des Trägermaterials. Aufgrund seiner durchgehenden Makroporen ist das Innere für die umgehende Flüssigkeit frei zugänglich, so daß die Heranführung von abzubauendem Material und Fortführung von Stoffwechselprodukten nicht gehemmt ist. Die feinporige Zerklüftung der Porenwände des Sintermaterials begünstigt dabei die Immobilisierung der Biomasse bzw. Mikroorganismen.

Angestrebt wird eine möglichst hohe Porosität des Materials, die noch mit ausreichender mechanischer Stabilität vereinbar ist. Aus diesem Grunde scheinen Träger mit einer Porosität über 85 % nicht mehr brauchbar zu sein. Porositäten unter 35 % sind ebenfalls uninteressant. Bevorzugt werden Porositäten über 40 %, die insbesondere zwischen 50 und 70 %, speziell bei etwa 55 bis 65 % liegen.

Der Porendurchmesser der Makroporen kann je nach Anwendungsbedingungen gewählt werden und wird üblicherweise nicht über 500 $\mu$m liegen. Als besonders zweckmäßig haben sich Makroporen im Bereich von 20 bis 250 $\mu$m, insbesondere 50 bis 150 $\mu$m, erwiesen. Als Durchmesser der Mikroporen sind im allgemeinen etwa 1 bis 10 $\mu$m geeignet. Der Beitrag der Mikroporen zum Gesamtporenvolumen kann üblicherweise etwa zwischen 5 und 15 % liegen je nach Größenverhältnis der Mikro- und Makroporen zueinander und Gesamtporosität, wobei hohe Porositäten aus mechanischen Gründen nur noch geringe Mikroporenanteile zu-lassen.

Das Material der Trägerkörper braucht nicht einheitlich zu sein, solange es ausreichend sinterfähig ist. Bevorzugt werden Glas, Keramik oder Glaskeramik, insbesondere silikatisches Material.

Weitere Besonderheiten der Erfindung gehen aus den Patentansprüchen hervor.

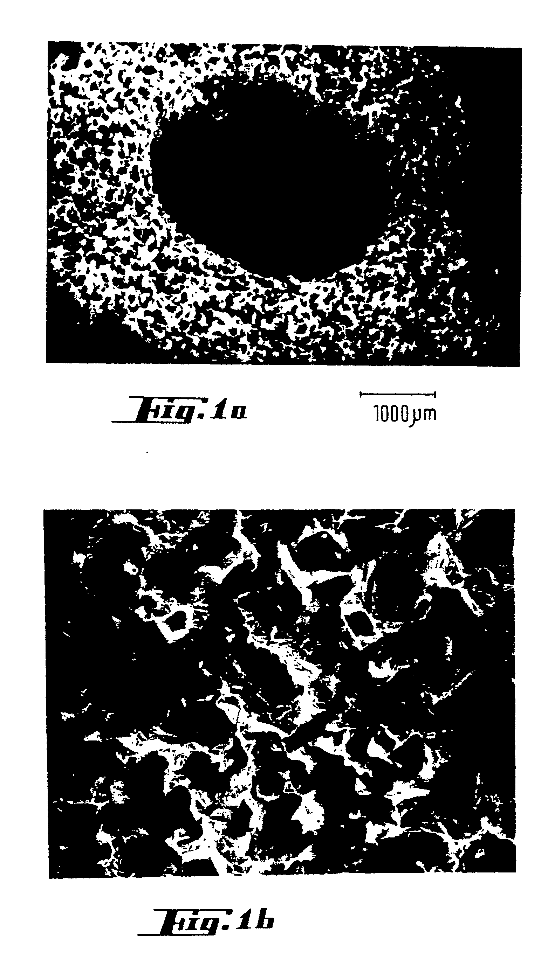

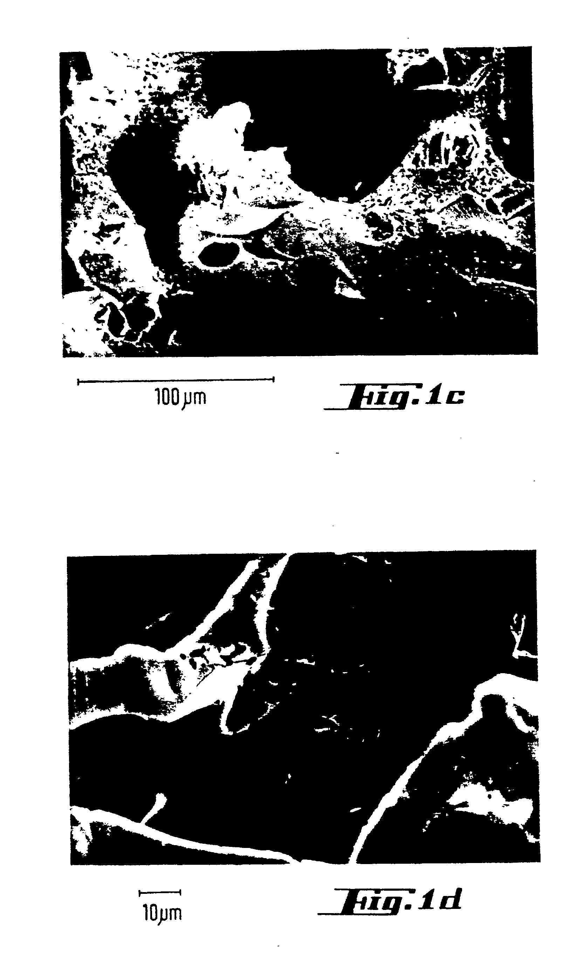

Die beigefügten elektronenmikroskopischen Vergrößerungen einer Glasstruktur zeigen den vorteilhaften Aufbau des Materials. Dabei zeigt Fig. 1a die Oberfläche eines Raschigring-Sinterglaskörper (19-fache Vergrößerung);

Fig. 1b die Schnittfläche eines solchen Körpers mit Porosität von 60 % und einer Porengröße der Makroporen von 60 bis 100 $\mu$m (104-fache Vergrößerung);

Fig. 1c die Schnittfläche eines solchen Körpers in 512-facher Vergrößerung

Fig. 1d die Schnittfläche eines solchen Körpers in 2000-facher Vergrößerung

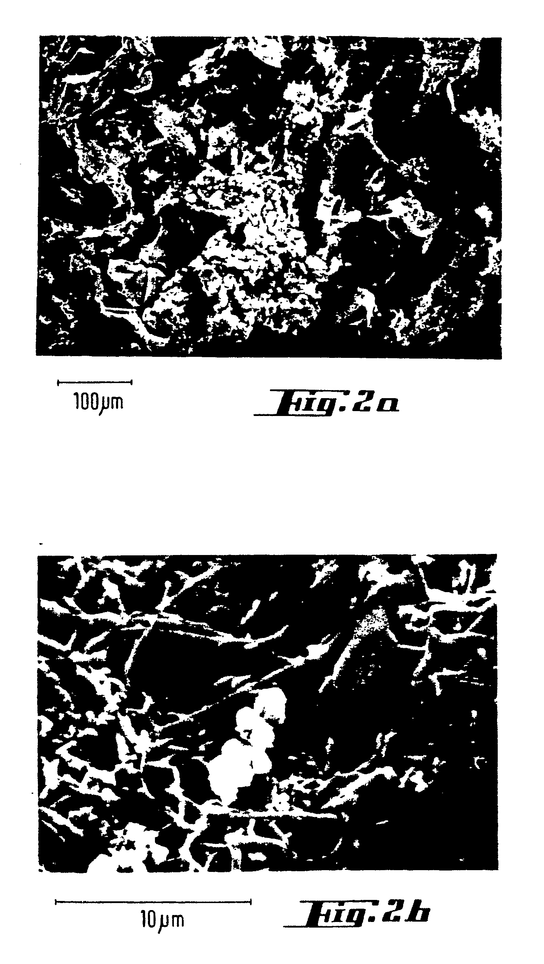

Fig. 2a und 2b mit Mikroorganismen bewachsener Sinterglaskörper nach 4-monatigem Reaktorbetrieb (200-fache bzw. 5040-fache Vergrößerung)

Fig. 3 schematische Darstellung eines Festbettumlaufreaktors

Fig. 4 Diagramm des Abwasserdurchsatzes in Abhängigkeit von der Betriebszeit
Fig. 5 Diagramm der Biogasproduktion in Abhängigkeit von der .Betriebszeit.

Das poröse Trägermaterial kann erhalten werden durch Versintern einer Pulvermischung, die aus einem feinkörnigen, sinterfähigen Material und einer etwas grobkörnigeren, höher als die Sintertemperatur schmelzenden, aus dem Sintermaterial herauslösbaren Substanz besteht, Abkühlen lassen und Herauslösen der löslichen Komponenten. Als herauslösbave Substanz wird vorzugs weise $\mu$m Salz verwendet.

Das Porenvolumen und der mittlere Porendurchmesser der Makroporen wird im wesentlichen durch die Menge an herauslösbarer Substanz und die Körnung der herauslösenden Substanz bestimmt. Der Porendurchmesser der Mikroporen, die nach dem Herauslösen der löslichen Substanz noch die Wände des (silicatischen) Sintergerüstes durchziehen, werden durch die Körnung des sinterfähigen Materials bestimmt. Makroporen die eine Größe von 20 bis 500 $\mu$m aufweisen, sind durch Verwendung einer aus dem Sinterprodukt herauslösbaren Substanz, vorzugsweise einer salzes mit einer Körnung von 20 - 600 $\mu$m und Mikroporen, die eine Größe von 1 - 10 $\mu$m aufweisen, sind durch Verwendung eines sinterfähigen Materials mit Korngröße < 40 $\mu$m, vorzugsweise < 20 $\mu$m erhältlich.

Der Vorteil dieses Verfahrens für die Herstellung von Trägermaterial für Bioreaktoren besteht darin, daß gleich-zeitig sehr feine und grobe Poren entstehen; während die Mikroporen in den Wänden für den Flüssigkeitsdurchtritt zu fein sind und sich auf Grund ihrer geringen Größe für die Immobilisierung der Mikroorganismen anbieten, gewährleisten die Makroporen die rasche Heranführung von Nährstoffen und die Fortführung der Stoffwechselprodukte.

Die gemäß der DE-OS 28 39 580 verwendeten Glasfritten, die nach dem üblichen Verfahren hergestellt werden, enthalten nur feine oder grobe Poren. Die Porendurchmesser und das Porenvolumen werden bei den Verwendeten Glasfritten und Sintermaterialien nur durch die verwendete Korngröße des Sintermaterials bestimmt. So sind die Wände der großporigen Sinterkörper nicht feinporig zerklüftet.

Neben den besonders günstigen Immobilisierungseigenschaften des Trägers, bei dem hohe Biomassekonzentrationen von hoher Aktivität erreicht werden (bedingt durch die große innere Oberfläche und freie Zugänglichkeit der in den Hohlräumen fixierten Kolonien, die im wesentlichen innerhalb des Trägers vor Abrieb geschützt aufwachsen, so daß auch schlecht haftende Populationen angezüchtet werden können) und die gezielte Einstellbarkeit der Struktur durch die Herstellungsbedingungen (angepaßt an die zu immobilisierenden Mikroorganismen und Art und Fließbedingungen der Flüssigkeit, in der sie zur Anwendung kommen) zeichnen sich die porösen Sinterkörper durch mechanische Festigkeit, gute Benetzbarkeit sowie thermische Stabilität aus, so daß sie leicht sterilisierbar sind. Sie sind preisgünstig und in dar Zusammensetzung leicht zu variieren.

So können die porösen Sinterkörper aus beliebigen Materialien, insbesondere aus Gläsern, besonders preiswert aus Abfallgläsern erhalten werden. Für die hier betrachtete Verwendung erscheinen jedoch Gläser als besonders interessant, die biologisch wichtige Spurenstoffe wie Verbindungen der Elemente Nickel, Molybdän, Kupfer, Kobalt und desgleichen enthalten. Es ist bekannt, daß Glas trotz seines global inerten Verhaltens einem gewissen Ionenaustausch mit der Umgebung unterliegt, so daß sich solche im Material enthaltenen Spurenelemente förderlich auf das Verhalten der Mikroorganismen auswirken können.

Die erfindungsgemäße Immobilisierung von Mikroorganismen und tierischen Zellen ist in Anbetracht der hervorragenden Eigenschaften der mit immobilisiertem Biomaterial bewachsenen porösen Sinterkörper für alle biotechnologischen Prozesse zweckmäßig, bei denen ihre Bioaktivität und gleichzeitige Immobilisierung von Nutzen sind.

Besonders zweckmäßig erscheint zur Zeit ihr Einsatz für die anaerobe Abwasserreinigung, insbesondere von Spezialabwässern, wie solchen der Zellstoffindustrie oder der Käsebereitung oder auch von Abwässern aus der Stärkeherstellung und von Brauereiabwässern. Daneben kommen die erfindungsgemäß immobilisierten Mikroorganismen auch für die Denitrifikation von Wasser in Betracht.

Die biotechnologische Gewinnung von ernährungsessentiellen und pharmakologischen Substanzen ist als weiteres Anwendungsgebiet zu nennen, ferner die Gewinnung primärer Metabolite durch Vergärung. Solche an porösen Sintergläsern immobilisierten Mikroorganismen sind für Biotransformationen nützlich, die im industriellen Maßstab betrieben werden sollen, wie etwa Steroidumwandlungen oder dergleichen.

Die hervorragenden Eigenschaften der auf einen porösen Sinterkörper immobilisierten Mikroorganismen wurden zunächst an Sinterglas in einem vertikal von unten nach oben durchströmten Festbettreaktor mit überlagertem Flüssigkeitsumlauf durch pH-gesteuerte Teilrezyklierung nachgewiesen, und zwar wurde bei der Aufbereitung von Brüdenkondensat der Zellstofffabrikation durch Austausch von bislang benutztem grobkörnigen Anthrazit durch poröses Sinterglas mit einer Porosität von etwa 60 % und Porendurchmessern von 60 bis 120 $\mu$m in Form von Raschigringen mit einer Wandstärke von 2 mm und einer Höhe von 7 mm unter sonst gleichen Bedingungen eine Effektivitätssteigerung (Verkürzung der mittleren Verweilzeit für vergleichbare Reinigung) um einen Faktor von etwa 5 erreicht.

4

Die insbesondere für Prozesse in Festbettreaktoren mit durch Teilrezyklierung erhöhter Strömungsgeschwindigkeit der Flüssigkeit durch das Festbett nützliche Immobilisierung der Mikroorganismen durch poröse Sinterkörper mit Doppelporenstruktur ist aber selbstverständlich auch bei anderen Reaktortypen zweckmäßig, wie beispielsweise bei Festbettreaktoren ohne Teilrezyklierung oder horizontal durchströmten Festbetten, wobei je nach Strömungsbedingungen eine gewisse Anpassung der Makroporen nützlich sein kann, deren Größe mit abnehmender Relativgeschwindigkeit der Flüssigkeit bezogen auf den Träger zunehmen sollte.

Auch für Wirbelschichtreaktoren eignet sich die erfindungsgemäße Immobilisierung der Mikroorganismen an porösen Sinterkörpern, die dann in geringerer Teilchengröße (< 1 mm) vorgesehen werden.

Bei Anwendung im sogenannten "Slurry Reaktor" (mit zwischen Filterwänden befindlicher feinteiliger Katalysatorsuspension) wird noch feinteiligeres Material eingesetzt:

Die große innere Oberfläche der porösen Sinterkörper mit frei zugänglichen Hohlräumen (mit feinporig zergliederten Hohlraumwänden), die von der Flüssigkeit frei durchströmt werden können, wodurch die Zellen ausreichend mit Nährstoff versorgt und Abbauprodukte fortgeführt werden können, während die feingliedrige Wandstruktur den Mikroorganismen ausreichend Gelegenheit für ein Anhaften bietet, ermöglicht die Verwendung größerer Trägerkörperformen in Festbetten, die zur Herabsetzung des Strömungswiderstandes beitragen. Besonders zweckmäßig sind Formen wie Raschigringkörper, die den Abtransport von Biogas aus dem Reaktor erleichtern.

Bei ihrem Einsatz werden die porösen Sinterkörper im trockenen Zustand mit der Zellsuspension kontaktiert, wobei die Mikroorganismen zusammen mit der Flüssigkeit in das Poreninnere gesaugt werden und dort an der feinporig zergliederten Wand Halt finden. Bei anaeroben Prozessen muß die in den Poren der Glaskörper vorhandene Luft zunächst durch Evakuieren oder Verdrängen durch Inertgas entfernt werden, um eine Vergiftung der Zellen durch den Sauerstoff zu vermeiden.

Mit fortschreitender Reaktionszeit wird die anfangs noch unbeladene Oberfläche der Sinterkörper bewachsen unter Ausbildung eines mikrobiellen Rasens, der u.a. an der veränderten,Farbe deutlich zu erkennen ist.

Beispiel 1

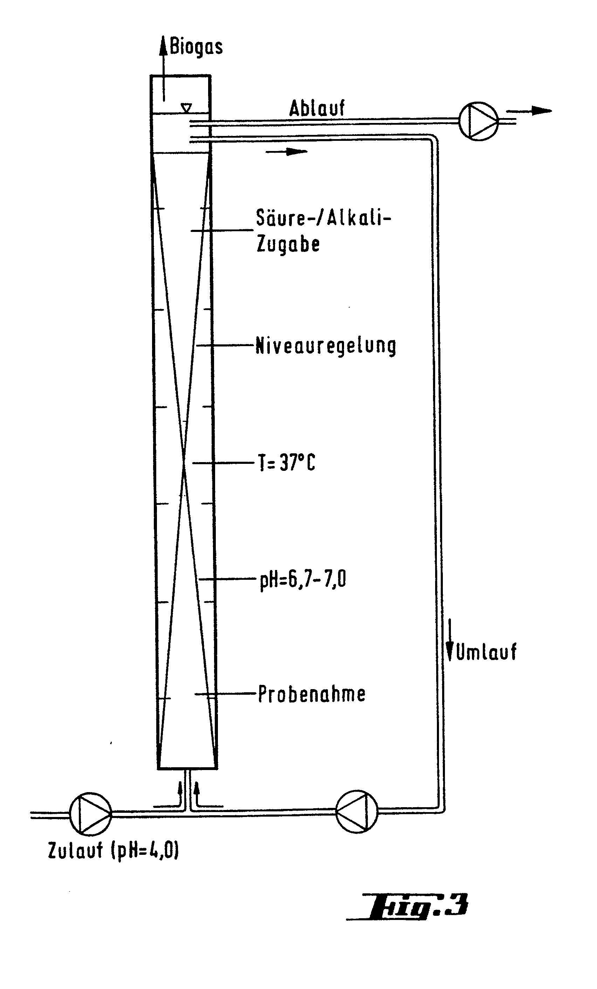

In einen Festbett-Umlaufreaktor wie in Figur 3 mit einer Höhe von 1,2 m, einem Durchmesser von 0,12 m und einem Arbeitsvolumen von 12 1 wurden 7,4 1 Sinterglaskörper in Würfelform (Kantenlänge: 0,5 cm) gefüllt. Die Porösität dieser Körper betrug 60 % bei einer Makroporengröße von 60 bis 100 $\mu$m. Die Mikroporengröße lag im Mittel bei 1 - 2 $\mu$m.

Zum Anfahren der Reaktion wurde die in den Poren der Glaskörper eingeschlossene Luft durch Hindurchleiten von Argon durch die Schüttung entfernt. Dann wurde eine auf die Inhaltsstoffe eines mit Schmutzstoffen extrem hoch belasteten Brüdenkondensats der Zellstoffindustrie adaptierte Mikroorganismensuspion mit 700 mg Trockensubstanz pro Liter in einer zur Füllung des Reaktors geeigneten Menge in den Reaktor eingelassen. Danach wurde die Abwasserzufuhr gestartet. Der pH-Wert wurd im unteren und oberen Teil des Reaktors überwacht und der Rezyklierungsanteil automatisch auf eine maximale pH-Wert-Differenz von 0,3 pH Einheiten kontrolliert, wie in der DE-Patentanmeldung P 33 45 691.7 beschrieben.

Diese Arbeitsweise gestattet, daß jeweils eine solche Menge Abwasser eingeleitet wird, die von den Mikroorganismen abgebaut werden kann und hat den Vorteil, daß der Anfahrvorgang schonend (ohne Gefahr einer Übersäuerung), jedoch gleichzeitig unter genügender Streßbelastung für die Mikroorganismen erfolgt.

Zu Beginn wurde mit einer Verweilzeit von 180 Std. gearbeitet. Daran anschließend wurde dann die iterative Verkürzung der Abwasser-Verweilzeit im Reaktor vom pH-Regler übernommen.

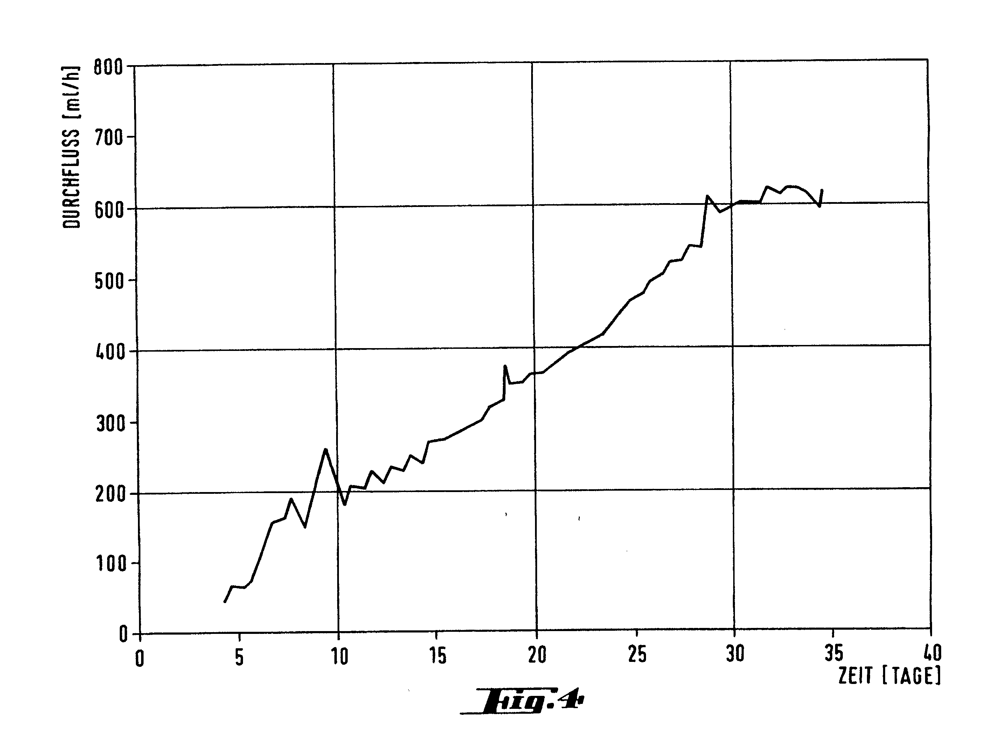

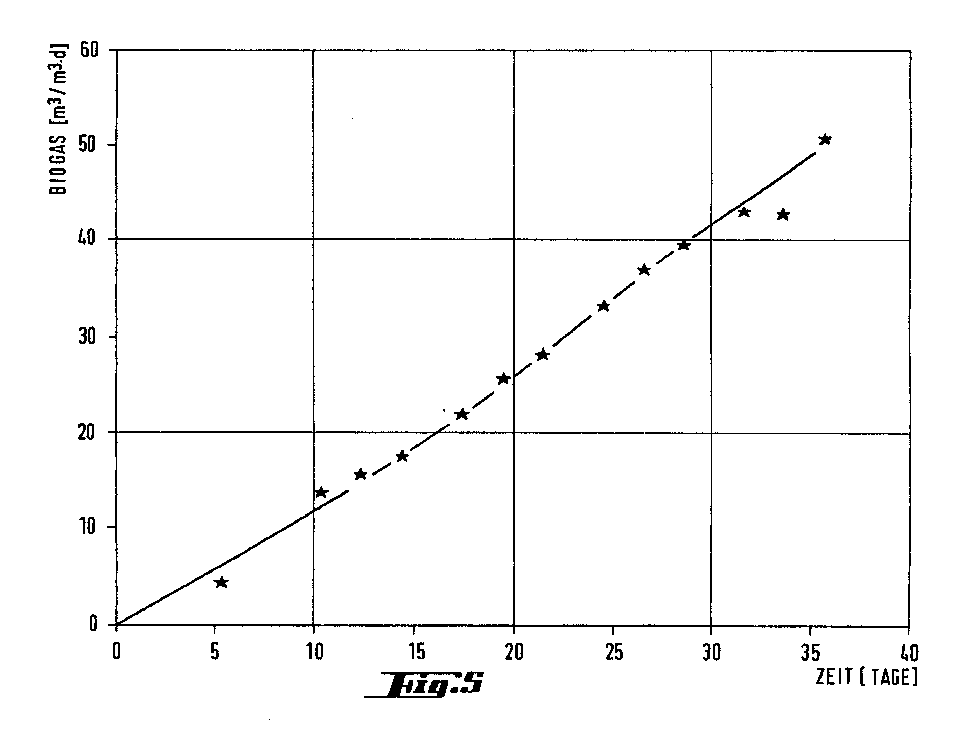

Figur 4 zeigt die lineare Steigerung des Abwasserdurchsatzes beim Anfahren des Reaktors in Abhängigkeit von der Betriebszeit. In Figur 5 ist die zugehörige Biosgasproduktion als Funktion der Zeit aufgetragen. Man beobachtet eine ebenfalls lineare Zunahme der Biogasbildung (Verdopplung in etwa 5,5 Tagen), die bei einer Verweilzeit von 12 Stunden einen Wert von 51 m$^3$/m$^3$ Reaktorvolumen und Tag erreicht. Dabei wurden bei einer Raumbelastung von 88 kg-CSB/(m$^3$d) 74 kg-CSB/(m$^3$d) eliminiert.

Die Versuchsergebnisse sind in Tabelle 1 am Ende der Beschreibung zusammengestellt.

Beispiel 2

In diesem Fall wurden ca. 8 1 eines porösen Sinterglasträgers in Form von Raschigringen (Wandstärke:

5

2 mm; Höhe: 7 mm) mit Makroporen von 60 - 100 $\mu$m und Mikroporen von 1 - 2 $\mu$m in einen Reaktor der gleichen Größe wie in Beispiel 1 eingefüllt. Der Reaktor wurde in der beschriebenen Weise vorbereitet und mit einer vergleichbar aktiven Mikroorganismensuspension zur Verarbeitung eine Brüdenkondensats wie in Beispiel 1 gefüllt. Hierbei zeigt sich, daß der Anstieg des Abwasserdurchflusses und der Biogasbildung unter sonst gleichen Bedingungen wie in Beispiel 1 langsamer erfolgt. Dies korreliert mit der geringeren Packungsdichte der Raschigringe im Vergleich zu den Würfeln. Nach etwa 6 Wochen Betrieb wurde allerdings auch mit der Raschigring-Füllung eine vergleichbar hohe Raumzeitausbeute erreicht. Insgesamt erweist sich dabei die Füllung mit raschigringförmigen Trägermterialien als günstiger im Vergleich zu würfelförmigen Trägerkörpern, da die Ringschüttung für die Durchströmung des Festbetts und den Gasaustrag zweckmäßiger sind.

Beispiel 3

Bei einem weiteren Versuch wurden 7 1 Glasträger-Material in Raschigringform wie in Beispiel 2 in die vorstehend beschriebene Versuchsapparatur eingefüllt. Zur Untersuchung gelangt in diesem Fall ein Abwasser aus dem Brauereigewerbe, welches beim Spülen von Fässern anfällt ("Fassreiniger"). Dieses Abwasser enthält vornehmlich Zucker, Essigsäure und Äthanol. Als bakterielles Inokulum diente eine zuvor auf diese Inhaltsstoffe adaptierte Bakterienmischkultur.

Die "Fassreiniger" sind relativ "magere" Abwässer mit einem chemischen Sauerstoffbedarf von 2,5 - 3,5 kg/m$^3$.

Der Reaktor wurde in der vorstehend beschriebenen (pH-Regler unterstützten) Betriebsweise angefahren und der Vorgang der iterativen Verweilzeitverkürzung anhand von Essigsäure- und CSB-Bestimmungen verfolgt.

Nach 5-wöchigen Betrieb wurde eine Verweilzeit von 7 Stunden erreicht, wobei gleichzeitig der chemische Sauerstoffbedarf im Ablauf um 94 % reduziert war.

Eine Zusammenstellung der wichtigsten Abbaudaten für diesen Versuch geht aus der nachfolgenden Tabelle hervor.

| | | |
|---|---|---|
| Verweilzeit (t) | : | 7,6 h |
| $CSB_o$ (Zulauf) | : | 2,74 kg/m$^3$ |
| $CSB_e$ (Ablauf) | : | 0,21 kg/m$^3$ |
| Raumbelastung | : | 10,53 kg CSB/(m$^3 \cdot$ d) |
| CSB-Umsatz | : | 93,5 % |
| $\Delta$ CSB | : | 9,85 kg/(m$^3 \cdot$ d) |
| Biogasbildung | : | 7,0 m$^3$/(m$^3 \cdot$ d) |

Tabelle    Anaerober Abbau des "Fassreinigers"

Die relativ geringe Verweilzeit von 7,6 Stunden im Reaktor ist gleichbedeutend mit einer hohen Raum/Zeit-Ausbeute und dies bei einem hervorragenden CSB-Umsatz von 93,5%. Dieses für den anaeroben Abbau von "magerem" Abwasser hervorragende Ergebnis läßt die günstige Wirkung der auf dem porösem Sinterglaskörper immobilisierten Biomasse klar erkennen.

6

Tabelle 1: Anaerober Abbau eines Brüdenkondensates im Festbett-Umlaufreaktor mit Sinterglas als Trägermaterial (Würfelform, a = 0,5 cm)

| | |
|---|---|
| Reaktorvolumen | : 12 1 |
| Trägerschüttung | : 7,4 1 |
| $CSB_{ein}$ (Fracht Zulauf) | : 44,0 $kg/m^3$ |
| $CSB_{aus}$ (Fracht Ablauf) | : 7,0 $kg/m^3$ |
| Verweilzeit | : 12 h |
| Biomassekonzentration | : 12,44 G/l (Aus Stickstoff-analyse) |
| CSB-Umsatz | : 84 % |
| Raumbelastung | : 88,0 $kg\text{-}CSB/(m^3 \cdot d)$ |
| Schlammbelastung | : 7,0 $kg\text{-}CSB/(kg \cdot d)$ |
| CSB-Elimination | : 74,0 $kg(m^3 \cdot d)$ |
| Schlammaktivität | : 5,9 $kg\text{-}CSB/(kg \cdot d)$ |
| Biogasbildung | : 51,0 $m^3/(m^3 \cdot d)$ |

**Ansprüche**

1. Verfahren zur Immobilisierung von Mikroorganismen und tierischen Zellen, insbesondere für anaerobe Prozesse, an porösen anorganischen Trägerkörpern, dadurch gekennzeichnet; daß man als Trägerkörper poröse Sinterkörper mit einer Porendoppelstruktur verwendet mit porositätsbestimmenden durchgehenden Makroporen, die einen freien Flüssigkeits- und Gasaustausch vom Trägerinneren zur Umgebung hin zulassen, und mit die Makroporenwände durchsetzenden offenen Mikroporen, deren Größe im Bereich der Mikroorganismen- bzw. Zellgröße liegt.

2. Verfahren nach Anspruch 1, dadurch gekennzeichnet, daß als Trägerkörper ein silikatischer Trägerkörper verwendet wird.

3. Verfahren nach Anspruch 1 und 2, dadurch gekennzeichnet, daß als anorganischer Trägerkörper ein Sinterglas verwendet wird.

4. Verfahren nach einem der vorangehenden Ansprüche, dadurch gekennzeichnet, daß ein Trägerkörper

EP 0 155 669 B1

verwendet wird, der ein offenes Porenvolumen von 35 % bis 85 % aufweist mit 20 bis 80 % Makroporen von 20 bis 500 $\mu$m Porendurchmesser und 15 - 5 % Mikroporen von 1 - 10 $\mu$m Porendurchmesser.

5. Verfahren nach Anspruch 4, dadurch gekennzeichnet, daß das gesamte offene Porenvolumen zwischen 50 und 70 %, vorzugsweise zwischen 55 und 65 % liegt, und der Anteil an Mikroporen < 10 $\mu$m 10 % bis 5 % beträgt.

6. Verfahren nach Anspruch 4 oder 5, dadurch gekennzeichnet, daß der mittlere Porendurchmesser der Makroporen bei 50 bis 150 $\mu$m liegt.

7. Verfahren nach einem der vorgenannten Ansprüche, dadurch gekennzeichnet, daß man Trägerkörper in Form von Raschigringen verwendet.

8. Verfahren nach einem der vorangehenden Ansprüche, dadurch gekennzeichnet, daß man Trägerkörper aus einem Material verwendet, das biologisch wichtige Spurenstoffe enthält.

9. Verfahren nach einem der Ansprüche 1 - 8, dadurch gekennzeichnet, daß es in einem Festbett-Umlaufreaktor durchgeführt wird.

10. Verfahren nach einem der Ansprüche 1 bis 8, dadurch gekennzeichnet, daß es in einem Wirbelbett-Reaktor durchgeführt wird.

11. Verfahren nach einem der vortergehenden Ansprüche, dadurch gekennzeichnet, daß es bei der anaeroben Reinigung von Abwässern der Papier- und Celluloseindustrie eingesetzt wird.

12. Verfahren nach einerm der Ansprüche 1 bis 10 dadurch gekennzeichnet, daß es zum anaeroben Abbau von Stärkeabwässern und Brauereiabwässern eingesetzt wird.

13. Verfahren nach einem der Ansprüche 1 bis 10, dadurch gekennzeichnet, daß es zur biotechnologischen Gewinnung von Substanzen eingesetzt wird.

14. Verfahren nach Anspruch 13, dadurch gekennzeichnet, daß es zur Gewinnung von ernährungsessentiellen und pharmakologischen Substanzen eingesetzt wird.

15. Verfahren nach Anspruch 14, dadurch gekennzeichnet, daß es zur Gewinnung von Gärungsprodukten eingesetzt wird.

16. Mit Mikroorganismen oder Zellmaterial bewachsener Trägerkörper, gekennzeichnet durch einen porösen Trägerkörper mit einer Porendoppelstruktur mit porositätsbestimmenden durchgehenden Makroporen, die einen freien Flüssigkeits-und Gasaustausch vom Trägerinneren zur Umgebung hin zulassen, und mit die Makroporenwände durchsetzenden offenen Mikroporen, deren Größe im Bereich der Mikroorganismen- bzw. Zellgröße liegt.

17. Trägerkörper zu Immobilisierung von Mikroorganismen in Form eines porösen anorganischen Sinterkörpers, gekennzeichnet durch eine Porendoppelstruktur mit porositätsbestimmenden durchgehenden Makroporen, die einen freien Flüssigkeits- und Gasaustausch vom Trägerinneren zur Umgebung hin zulassen, und mit die Makroporenwände durchsetzenden offenen Mikroporen, deren Größe im Bereich der Mikroorganismen- bzw. Zellgröße liegt.

18. Trägerkörper nach Anspruch 17, dadurch gekennzeichnet, daß die Makroporen eine Größe von 20 bis 500 $\mu$m aufweisen.

19. Trägerkörper nach Anspruch 17, dadurch gekennzeichnet, daß die Mikroporen eine Größe von 1 bis 10 $\mu$m aufweisen.

**Claims**

8

1. A method for the immobilization of micro-organisms and animals cells, especially for anaerobic processes, at porous inorganic carrier bodies, characterized in that porous sintered bodies comprising a double porous structure are used as carrier bodies, said porous sintered bodies having porosity-determining through-passing macro-pores which grant a free fluid and gas exchange from the inside of the carriers to the environment and having open micro-pores which intersperse the macro-pores, the size of said micro-pores being in the range of the size of micro-organisms or cells respectively.

2. A method according to claim 1, characterized in that for a carrier body a silicious carrier body is used.

3. A method according to claim 1 and 2, characterized in that for an inorganic carrier body a sintered glass is used.

4. A method according to anyone of the proceeding claims, characterized in that a carrier body is used having an open pore volume of 35 to 85% and comprising 20 to 80% macro-pores with pore diameters in the range of 20 to 500 $\mu$m and 15 to 5% micro-pores with pore diameters in the range of 1 to 10 $\mu$m.

5. A method according to claim 4, characterized in that the whole open pore volume lies between 50 and 70%, preferably between 55 and 65% and that the amount of micro-pores being smaller than 10 $\mu$m lies between 10% and 5%.

6. A method according to claims 4 or 5, characterized in that the medium pore diameter of the macro-pores lies in a range between 15 and 150 $\mu$m.

7. A method according to any one of the preceeding claims, characterized in that carrier bodies in the form of Raschig rings are used.

8. A method according to anyone of the preceeding claims, characterized in that for the carrier bodies a material containing biologically essential trace elements is used.

9. A method according to anyone of claims 1 to 8, characterized in that it is executed in a fixed-bed circulating reactor.

10. A method according to anyone of claims 1 to 8, characterized in that it is executed in a fluidized-bed reactor.

11. A method according to any one of the preceeding claims, characterized in that it is employed in anaerobic waste water purification of the paper and cellulosis-processing industry.

12. A method according to any one of the claims 1 to 10, characterized in that it is employed to the anaerobic desintegration of starch containing waste water or of waste water of the brewery industry.

13. A method according to any one of the claims 1 to 10, characterized in that it is employed for the biotechnological production of substances.

14. A method according to claim 13, characterized in that it is employed for the production of nutritionally essential and pharmacological substances.

15. A method according to claim 14, characterized in that it is employed for the production of fermentation products.

16. A carrier body covered by micro-organisms or cell material, characterized by a porous carrier body comprising a double porous structure with porosity-determining through-passing macro-pores which grant a free fluid and gas exchange from the inside of the carriers to the environment, said carrier bodies having open micro-pores which intersperse the macro-pores, the size of said macro-pores being in the range of the size of the micro-organisms or the cells.

17. A carrier body for the immobilization of micro-organisms in the form of a porous inorganic sintered body, characterized by a double-porous structure with porosity-determining through-passing macro-pores which grant a free fluid and gas exchange from the inside of the carrier bodies to the environment, said carrier body having open micro-pores which intersperse the walls of the macro-pores, the size of the micro-pores being in the range of the size of the micro-organisms or the cells.

18. A carrier body according to claim 17, characterized in that the macro-pores have a size in the range of 20 to 500 $\mu$m.

19. A carrier body according to claim 17, characterized in that the micro-pores have a size in the range of 1 to 10 $\mu$m.


**Revendications**

1. Procédé pour l'immobilisation de micro-organismes et de cellules animales, notamment pour des processus anaérobies, sur des supports non organiques poreux, caractérisé en ce qu'on utilise comme supports des corps frittés poreux avec une structure de pore double comportant des macropores traversants déterminant la porosité, qui permettent un échange libre de liquide et de gaz de l'intérieur du support avec l'environnement, et des micropores ouverts traversant les parois des macropores dont la taille est de l'ordre de celle des micro-organismes ou des cellules.

2. Procédé selon la revendication 1, caractérisé en ce qu'on utilise comme support un support à base de silicate.

3. Procédé selon les revendications 1 et 2, caractérisé en ce qu'on utilise comme support non organique un verre fritté.

4. Procédé selon l'une des revendications précédentes, caractérisé en ce qu'on utilise un support qui présente un volume de pores ouverts compris entre 35 % et 85 % avec 20 à 80 % de macropores avec un diamètre de pores compris entre 20 et 500 $\mu$m et avec 15 à 5 % de micropores de diamètre de pores compris entre 1 et 10 $\mu$m.

5. Procédé selon la revendication 4, caractérisé en ce que le volume total de pores ouverts est compris entre 50 et 70 %, de préférence entre 55 et 65 % et en ce que la proportion de micropores inférieurs à 10 $\mu$m est comprise entre 10 et 5 %.

6. Procédé selon la revendication 4 ou 5, caractérisé en ce que le diamètre moyen des pores des macropores est compris entre 50 et 150 $\mu$m.

7. Procédé selon l'une des revendications précédentes, caractérisé en ce qu'on utilise des supports en forme d'anneaux de Raschig.

8. Procédé selon l'une des revendications précédentes, caractérisé en ce qu'on utilise des supports réalisés en un matériau qui contient des éléments traces importants sur le plan biologique.

9. Procédé selon l'une des revendications 1 à 8, caractérisé en ce qu'il est appliqué dans un réacteur à circulation à lit fixe.

10. Procédé selon l'une des revendications 1 à 8, caractérisé en ce qu'il est appliqué dans un réacteur à lit fluidisé.

11. Procédé selon l'une des revendications précédentes, caractérisé en ce qu'il est appliqué pour l'épuration anaérobie des eaux usées de l'industrie du papier et de la cellulose.

12. Procédé selon l'une des revendications 1 à 10, caractérisé en ce qu'il est appliqué pour la décomposition anaérobie des eaux usées de l'industrie de l'amidon et des brasseries.

13. Procédé selon l'une des revendications 1 à 10, caractérisé en ce qu'il est appliqué pour la récupération par voie biotechnologique de substances.

14. Procédé selon la revendication 13, caractérisé en ce qu'il est appliqué pour la production des substances essentielles dans l'alimentation et de substances pharmacologiques.

15. Procédé selon la revendication 14, caractérisé en ce qu'il est utilisé pour la production de produits de fermentation.

16. Support ensemencé de micro-organismes ou de matériaux cellulaires caractérisé par un corps-support poreux comportant une structure à pores double avec des macropores traversants déterminant la porosité, qui permettent un échange libre de liquide et de gaz de l'intérieur du support avec l'environnement, et des micropores ouverts traversant la paroi des macropores dont la taille est de l'ordre de la taille des micro-organismes ou des cellules.

17. Support pour l'immobilisation de micro-organismes sous forme d'un corps fritté non organique poreux caractérisé par une structure à pores double avec des macropores traversants déterminant la porosité, qui permettent un échange libre de liquide et de gaz de l'intérieur du support avec l'environnement, et des micropores ouverts traversant la paroi des macropores dont la taille est de l'ordre de la taille des micro-organismes et des cellules.

18. Support selon la revendication 17, caractérisé en ce que les macropores ont une taille comprise entre 20 et 500 $\mu$m.

19. Support selon la revendication 17, caractérisé en ce que les micropores ont une taille comprise en 1 et 10 $\mu$m.

Fig.1a

1000µm

Fig.1b

100 μm

*Fig. 1c*

10 μm

*Fig. 1d*

100μm   *Fig.2a*

10μm   *Fig.2b*

14

**↑ Biogas**

**Ablauf**

Säure-/Alkali-
Zugabe

Niveauregelung

T= 37°C

pH=6,7-7,0

↓Umlauf

Probenahme

Zulauf (pH=4,0)

**Fig.3**

Fig.4

EP 0 155 669 B1

Fig.5